# EUROPÄISCHE PATENTANMELDUNG

(11) **EP 2 080 515 A1**
(43) Veröffentlichungstag der Anmeldung: **22.07.2009**
(21) Anmeldenummer: 09006259.7
(22) Anmeldetag: 30.10.2004
(51) Int. Cl.: A61K 31/495

(54) **Geschmacksstoffhaltige Arzneimittelformulierungen mit verbesserten pharmazeutischen Eigenschaften**

(30) Priorität: 04.11.2003 DE 10351448
(62) Teilanmeldung aus: 04791080.7
(71) Anmelder: Bayer Animal Health GmbH, 51368 Leverkusen (DE)
(72) Erfinder: Bosché, Patrick, 79639 Grenzach-Whylen (DE); Bongaerts (née Becker), Sabine, 41541 Dormagen (DE); Kanikanti, Venkata-Rangarao, 51381 Leverkusen (DE)

(57) **Zusammenfassung**

Die Erfindung betrifft geschmacksstoffhaltige Arzneimittelformulierungen mit verbesserten pharmazeutischen Eigenschaften sowie Verfahren zu deren Herstellung.

## Beschreibung

Die Erfindung betrifft geschmacksstoffhaltige Arzneimittelformulierungen mit verbesserten pharmazeutischen Eigenschaften sowie Verfahren zu deren Herstellung.

Die Verwendung von Aromastoffen in Arzneimittelformulierungen gewinnt auf dem veterinärmedizinischen Sektor immer mehr an Bedeutung. Die Aromastoffe sollen die Applikation fester Arzneiformen an Tieren, beispielsweise Hunde oder Katzen erleichtern. Im Markt befindliche Aromastoffe, wie z.B. Trigarol Bayopal P^{®} (Haarmann und Reimer GmbH, Holzminden, D) oder Artificial Beef Flavor (Pharma Chemie, Syracuse, Nebraska, USA) haben einen negativen Einfluss auf die pharmazeutischen Eigenschaften von Tabletten. So wird z.B. die Tablettenhärte (Bestimmung der Tablettenhärte z.B. beschrieben in Bauer, Kurt H.; Frömming, Karl-Heinz; Führer, Claus: Lehrbuch der Pharmazeutischen Technologie, 6., durchges. u. korr. Aufl. 1999) herabgesetzt oder die Stabilität der Tabletten wird durch verschlechterte Freisetzung oder Tablettenhärte negativ beeinflusst. Durch diese Änderungen ergeben sich Nachteile für die Qualität der pharmazeutischen Produkte.

Botzolakis, Harris und Nesbitt beschreiben in Pharm. Res. (5, No. 10, Suppl. S253, 1988) und in EP-A-0345787 bzw. US 04910023 Formulierungen mit schlecht schmeckenden Wirkstoffen und Verfahren zu ihrer Herstellung. Dabei werden schlecht schmeckende, hygroskopische Wirkstoffe in Wasser aufgeschlämmt und in Kombination mit hochdispersem Siliciumdioxid getrocknet. Dabei werden gut schmeckende, weniger hygroskopische Tabletten erhalten.

Die Anforderungen an eine feste Arzneimittelformulierung, geeignet für die Applikation bei Tieren, sind vielfältig:
- gute Akzeptanz durch die Tiere, im besten Fall freiwillige Aufnahme
- gute Lagerstabilität, insbesondere geringe Tendenz zur Wasseraufnahme
- gute mechanische Eigenschaften, insbesondere Tablettenhärte
- gute Zerfalls- und Freisetzungseigenschaften
   Die erhöhte Akzeptanz lässt sich durch Zusatz von Aroma- bzw. Geschmacksstoffen erreichen. Dabei bestand bislang das Problem, dass diese Aromastoffe die pharmazeutischen Eigenschaften der entsprechenden festen Formulierungen verschlechtern.
   Es wurde nun überraschenderweise gefunden, dass durch Zusatz von vergleichsweise hohen Mengen an hochdispersem Siliciumdioxid die nachteiligen Einflüsse der Geschmacks- bzw. Aromastoffe verringert oder völlig ausgeglichen werden können und man feste Arzneimittelformulierungen erhält, die in allen oben genannten Kriterien gute oder sehr gute Eigenschaften aufweisen. Dabei ist bemerkenswert, dass das hochdisperse Siliciumdioxid die Wirkung der Geschmacks- bzw. Aromastoffe kaum beeinflusst, obwohl es im Stand der Technik zur Geschmacksmaskierung vorgeschlagen wird.
   Die Erfindung betrifft daher feste Arzneimittelformulierungen, enthaltend einen pharmazeutischen Wirkstoff, einen Geschmacksstoff und mindestens 1,5 Gew.-% hochdisperses Siliciumdioxid bezogen auf das Gesamtgewicht der fertigen Formulierung.
   Die erfindungsgemäßen Arzneimittelformulierungen enthalten üblicherweise den pharmazeutischen Wirkstoff in einer Menge von 0,001 - 90 Gew.-%.
   Es kommen generell alle in der Veterinärmedizin üblichen pharmazeutischen Wirkstoffe in Frage. Als Beispiele seien genannt: Chinolon-Antibiotika sowie Mittel zur Behandlung von Krebserkrankungen, insbesondere MMP-Inhibitoren.
   Chinolon-Antibiotika sind unter anderem in folgenden Dokumenten beschrieben: US 4 670 444 (Bayer AG), US 4 472 405 (Riker Labs), US 4 730 000 (Abbott), US 4 861 779 (Pfizer), US 4 382 892 (Daiichi), US 4 704 459 (Toyama), als konkrete Beispiele für Chinolon-Antibiotika seien genannt: Ciprofloxacin, Enrofloxacin, Ibafloxacin, Sarafloxacin, Difloxacin, Binfloxacin, Danofloxacin, Marbofloxacin, Benofloxacin, Ofloxacin, Orbifloxacin, Tosufloxacin, Temafloxacin, Pipemidsäure, Norfloxacin, Pefloxacin, Ofloxacin, Fleroxacin. Weiterhin seien als geeignete Chinolonantibiotika die in WO 97/31001 beschriebenen Verbindungen genannt, insbesondere Pradofloxacin (8-Cyan-1-cyclopropyl-7-((1S,6S)-2,8-diazabicyclo[4.3.0]nonan-8-yl)-6-fluor-1,4-dihydro4-oxo-3-chinolincarbonsäure) der Formel

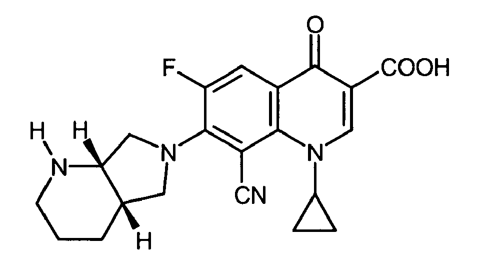

Bevorzugte Chinolon-Antibiotika werden allgemein durch die Formeln (I) und (II) dargestellt 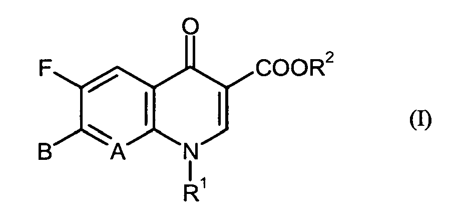 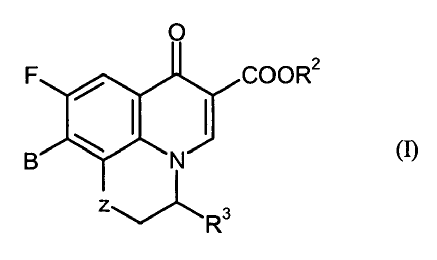 in denen
- A: für Stickstoff oder =C-R⁴ steht,
- R⁴: für Wasserstoff, Fluor, Chlor, Cyano, Nitro oder Methyl steht,
- B: für 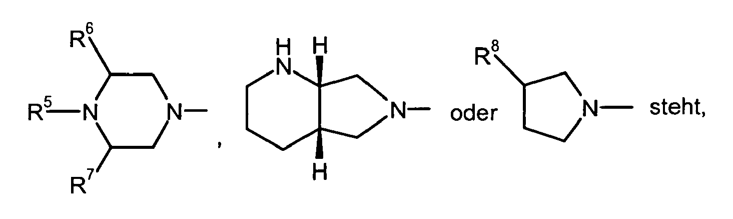
und
- R⁵: für Wasserstoff, eine verzweigte oder unverzweigte Alkylgruppe mit 1 bis 4 Kohlenstoffatomen, die gegebenenfalls durch eine Hydroxy- oder Methoxygruppe substituiert sein kann, steht,
- R⁶: für Wasserstoff, Methyl oder Phenyl steht,
- R⁷: für Wasserstoff oder Methyl steht,
- R⁸: für Amino, Alkyl- oder Dialkylamino mit 1 oder 2 Kohlenstoffatomen in der Alkylgruppe, Aminomethyl, Alkyl- oder Dialkylaminomethyl mit 1 oder 2 Kohlenstoffatomen in der Alkylgruppe steht,
- R¹: für einen Alkylrest mit 1 bis 3 Kohlenstoffatomen, Cyclopropyl, 2-Fluorethyl, Vinyl, Methoxy, 4-Fluorphenyl oder Methylamino steht,
- R²: für Wasserstoff, Alkyl mit 1 bis 6 Kohlenstoffatomen sowie Cyclohexyl, Benzyl, 2-Oxo-propyl, Phenacyl sowie Ethoxycarbonylmethyl steht,
- R³: für Wasserstoff, Methyl oder Ethyl steht,
- Z: für Sauerstoff, durch Methyl oder Phenyl substituierten Stickstoff sowie -CH₂- steht,
und ihre pharmazeutisch verwertbaren Salze.

Bevorzugt sind als Wirkstoffe Chinoloncarbonsäuren und ihre Derivate der Formel (Ia) 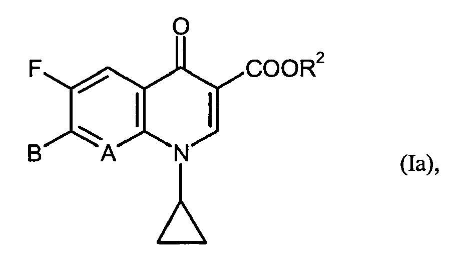 in welcher
- B: für 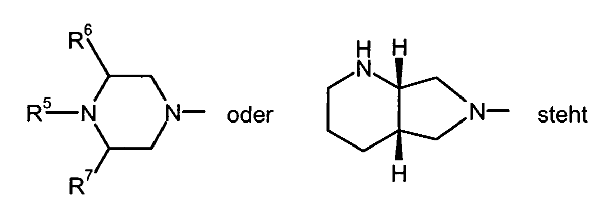
und
A, R², R⁵, R⁶ und R⁷ die oben angegebene Bedeutung haben.

Besonders bevorzugt sind als Wirkstoffe Chinoloncarbonsäure und ihre Derivate der Formel (Ia) 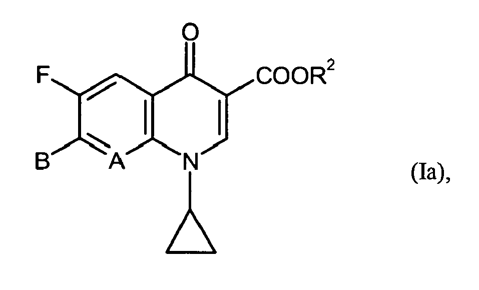 in welcher
- R²: für Wasserstoff, Alkyl mit 1 bis 4 Kohlenstoffatomen sowie Benzyl, 2-Oxopropyl, Phenacyl sowie Ethoxycarbonylmethyl steht,
- B: für 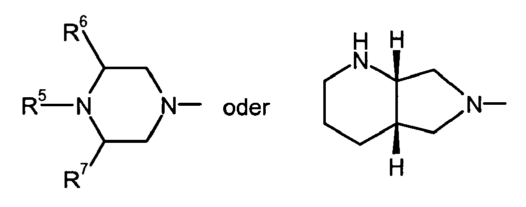 steht ,
- R⁵: für Wasserstoff, Methyl oder Ethyl steht,
- R⁶: für Wasserstoff oder Methyl steht,
- R⁷: für Wasserstoff oder Methyl steht und
- A: die oben angegebene Bedeutung hat.

Insbesondere seien als Wirkstoffe die folgenden Chinoloncarbonsäuren und ihre Derivate genannt:

1-Cyclopropyl-6-fluor-1,4-dihydro-4-oxo-7-(1-piperazinyl)-chinolin-3-carbonsäure (Ciprofloxacin), 1-Cyclopropyl-6-fluor-1,4-dihydro-4-oxo-7-(4-Methyl-1-piperazinyl)-chinolin-3-carbonsäure, 1-Cyclopropyl-6-fluor-1,4-dihydro-4-oxo-7-(4-Ethyl-1-piperazinyl)-chinolin-3-carbonsäure (Enrofloxacin), 1-Ethyl-6-fluor-1,4-dihydro-4-oxo-7-(4-methyl-1-piperazinyl)-chinolin-3-carbonsäure, 1-Ethyl-6-fluor-1,4-dihydro-4-oxo-7-(1-piperazinyl)-chinolin-3-carbonsäure, 1-Ethyl-6-fluor-1,4-dihydro-4-oxo-7-(1-piperazinyl)-1,8-naphthyridin-3-carbonsäure, 9-Fluor-3-methyl-10-(4-methyl-1-piperazinyl)-7-oxo-2,3-dihydro-7H-pyrido-[1,2,3-de]1,4-benzoxazin-6-carbonsäure und deren pharmazeutisch verwendbaren Salze sowie die Methyl- und Ethylester dieser Verbindungen.

Ganz besonders bevorzugt werden Enrofloxacin und Pradofloxacin sowie deren pharmazeutisch verwendbaren Salze eingesetzt.

Als pharmazeutisch verwendbare Salze seien physiologisch verträgliche Säureadditionssalze sowie Salze mit Basen genannt. Die Salze sind bekannt oder lassen sich analog zu bekannten Verfahren herstellen.

Als Säuren seien beispielsweise genannt: Salzsäure, Schwefelsäure, Phosphorsäure, organische Säuren wie Ameisensäure, Essigsäure, Milchsäure, Apfelsäure, Fumarsäure, Citronensäure, Ascorbinsäure, Bernsteinsäure, Weinsäure, Malonsäure, Maleinsäure, Embonsäure.

Bevorzugt seien genannt Salzsäure, Essigsäure, Milchsäure, Embonsäure.

Salze und Basen sind beispielsweise die Alkali-, Erdalkali-, Silber- und Guanidiniumsalze der zugrundeliegenden Carbonsäuren. Als geeignete Basen seien beispielhaft genannt anorganische Basen wie NaOH, KOH, Ca(OH)₂, Ammoniak, organische Basen wie Amine wie Mono-, Di-, Trialkylamine, substituierte Amine wie Ethanolamin, cyclische Amine wie Morpholin, Piperazin, basische Aminosäuren wie Arginin, Lysin, Cholin, N-Methylglucamin.

Bevorzugt sind die folgenden Basen: NaOH, KOH, Ethanolamin, Lysin, N-Methylglucamin.

Besonders bevorzugt sind die folgenden Basen: NaOH, KOH.

Pharmazeutisch annehmbare Solvate, insbesondere Hydrate der Wirkstoffe oder der Salze, etc. können ebenfalls eingesetzt werden.

Die Wirkstoffe sind bekannt oder lassen sich analog zu bekannten Verfahren herstellen.

Geeignete MMP-Inhibitoren sind in WO 96/15 096 ausführlich beschrieben, auf dieses Dokument wird ausdrücklich Bezug genommen. Unter den dort offenbarten Verbindungen sind solche mit Biphenyl-Grundstrukturen bevorzugt; als besonders bevorzugtes Beispiel sei die Verbindung Tanomastat genannt:

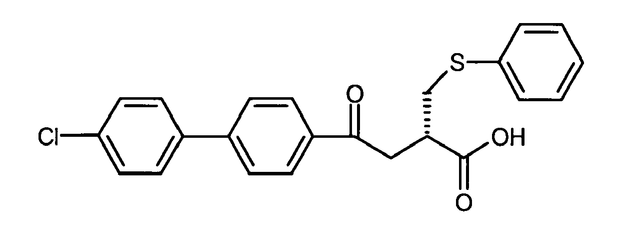

Pharmazeutisch verträgliche Salze, Hydrate etc. können ebenfalls eingesetzt werden.

Bei den erfindungsgemäß eingesetzten Geschmacks- bzw. Aromastoffen handelt es sich um Mischungen aus Proteinen, Fetten und Kohlenhydraten, die speziell aufgearbeitet werden. Insbesondere seien genannt Trigarol Bayopal P®, der Firma Haarmann & Reimer sowie Artificial Beef Flavor® der Firma Pharma Chemie (Syracuse, Nebraska, USA). In den erfindungsgemäßen Arzneimittelformulierungen werden die Geschmacks- bzw. Aromastoffe in einer Menge von 1 - 40 Gew.-%, bezogen auf das Gesamtgewicht der fertigen Formulierung, vorzugsweise 2,5 - 30 Gew.-%, insbesondere 4 - 20 Gew.-% eingesetzt.

Hochdisperses Siliciumdioxid ist z.B. in der Europäischen Pharmacopoe (Ph.Eur., SILICA, COLLOIDAL ANHYDROUS) oder dem Amerikanischen Arzneibuch (USP, Colloidal Silicon dioxide) beschrieben. Handelsprodukte sind z.B. Aerosil (Degussa), Dissolvurol oder Entero-Teknosal.

Die erfindungsgemäßen festen Arzneimittelformulierungen enthalten mindestens 1,5 Gew.-% hochdisperses Siliciumdioxid bezogen auf das Gesamtgewicht der fertigen Formulierung, bevorzugt enthalten sie mindestens 2,5 Gew.-%, besonders bevorzugt mindestens 4 Gew.-% hochdisperses Siliciumdioxid bezogen auf das Gesamtgewicht der fertigen Formulierung. Üblicherweise enthalten die erfindungsgemäßen festen Arzneimittelformulierungen nicht mehr als 15 Gew.-%, bevorzugt nicht mehr als 10 Gew.-% an hochdispersem Siliciumdioxid. Im Rahmen der vorstehend angegebenen Grenzen hat es sich als besonders vorteilhaft erwiesen, das hochdisperse Siliciumdioxid im Gewichtsverhältnis 1:4 bis 1:1 hochdisperses Siliciumdioxid zu Aromastoff zu verarbeiten.

Weiterhin wurde ein Verfahren zur Herstellung der erfindungsgemäßen festen Arzneimittelformulierungen gefunden, bei dem man den Geschmacksstoff mit dem hochdispersen Siliciumdioxid sowie einem oder mehreren Wirkstoffen und pharmazeutisch üblichen Hilfsstoffen granuliert. Für das erfindungsgemäße Herstellverfahren kommen folgende Varianten in Frage.
- Zusatz des Geschmacksstoffes und des hochdispersen Siliciumdioxids zur Nachmischung (d.h. zu einem Granulat bestehend aus den anderen Inhaltsstoffen), Mischen der festen Komponenten, gegebenenfalls nach Siebung beispielsweise durch ein Sieb von 0,5 - 2 mm Maschenweite, in einem geeigneten Behältnis für 2 - 30 Minuten.
- wässrige Granulation des Geschmacksstoffes mit dem hochdispersen Siliciumdioxid unter Verwendung von Bindemittellösungen bestehend aus Wasser oder Wasser mit beispielsweise Stärken (Maisstärke, Reisstärke, Weizenstärke, Kartoffelstärke), modifizierte Stärken (vorverkleisterte Stärke, Hydroxyethylstärke), Gelatine, Tragant, Cellulosederivaten (Methylcellulose, Hydroxypropylmethylcellulose, Hydroxypropylcellulose, Natrium-Carboxymethylcellulose) oder Polyvinylpyrrolidon, Zusatz dieses Granulates zur Nachmischung (d.h. zu einem Granulat bestehend aus den anderen Inhaltsstoffen) oder zur weiteren Granulation
- alkoholische (z.B. ethanolische) Granulation des Geschmacksstoffes mit dem hochdispersen Siliciumdioxid, Zusatz dieses Granulates zur Nachmischung oder zur weiteren Granulation
- Granulation des Geschmacksstoffes mit dem hochdispersen Siliciumdioxid unter Verwendung eines weiteren Bindemittels, Zusatz dieses Granulates zur Nachmischung oder zur weiteren Granulation
- Zusatz des Geschmacksstoffes mit dem hochdispersen Siliciumdioxid zur Granulation des Wirkstoffes eventuell unter Zusatz weiterer Hilfsstoffe wie z.B. Füllmittel, Zerfallhilfsmittel oder Bindemittel.

Die Granulation kann mittels Schnellmischer als Nassgranulation (abbauende Granulation) mit anschließender Trocknung beispielsweise im Wirbelschicht- oder Hordentrockner bei Zulufttemperaturen von 40 - 120°C erfolgen oder als aufbauende Granulation z.B. in einem Wirbelschichtgranulator.

Die erfindungsgemäßen Arzneimittelformulierungen eignen zum Einsatz in der Veterinärmedizin, z.B. in der Tierhaltung und in der Tierzucht. Sie können Nutz-, Zucht-, Zoo-, Labor-, Versuchs-und Hobbytieren verabreicht werden.

Zu den Nutz- und Zuchttieren gehören Säugetiere, wie z.B. Rinder, Pferde, Schafe, Schweine, Ziegen, Kamele, Wasserbüffel, Esel, Kaninchen, Dammwild, Rentiere, Pelztiere, wie z.B. Nerze, Chinchilla, Waschbär, Vögel, wie z.B. Hühner, Gänse, Puten, Enten, Tauben, sowie Vogelarten für Heim- und Zoohaltung. Ferner gehören dazu Nutz- und Zierfische.

Zu den Labor- und Versuchstieren gehören Mäuse, Ratten, Meerschweinchen, Goldhamster, Hunde und Katzen.

Zu den Hobbytieren gehören Hunde und Katzen.

Die erfindungsgemäßen festen Arzneimittelformulierungen sind vorzugsweise Tabletten. Es kommt jedoch auch andere feste Zubereitungen in Frage, wie Pulver, Premixe oder Konzentrate, Granulate, Pellets, Boli, Kapseln, Aerosole und Inhalate.

Die erfindungsgemäßen festen Arzneimittelformulierungen können weitere pharmazeutisch verträgliche Zusatz- und Hilfsstoffe enthalten, wie z.B. Trägerstoffe, sowie die weiter unten aufgeführten Hilfsstoffe.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, Phosphate.

Organische Stoffe sind z.B. Zucker, Milchzucker, Mannit, Zellulose, Nahrungs- und Futtermittel wie Milchpulver, Tiermehle, Getreidemehle und -schrote, Stärken.

Hilfsstoffe sind Antioxidantien, wie z.B. Ascorbinsäure, Tocopherol, Farbstoffe, die zur Anwendung am Tier zugelassen sind und gelöst oder suspendiert eingesetzt werden können.

Weitere geeignete Hilfsstoffe sind Schmier- und Gleitmittel wie z.B. Magnesiumstearat, Stearinsäure, Talkum, Bentonite, zerfallsfördemde Substanzen wie Stärke und Stärkederivate, Croscarmellose-Natrium oder quervernetztes Polyvinylpyrrolidon, Bindemittel wie z.B. Stärken (Maisstärke, Reisstärke, Weizenstärke, Kartoffelstärke), modifizierte Stärken (vorverkleisterte Stärke, Hydroxyethylstärke), Gelatine, Tragant, Cellulosederivaten (Methylcellulose, Hydroxypropylmethylcellulose, Hydroxypropylcellulose, Natrium-Carboxymethylcellulose) oder Polyvinylpyrrolidon sowie Trockenbindemittel wie mikrokristalline Cellulose.

Abbildungen:
Abbildung 1 zeigt die Abhängigkeit der Bruchfestigkeit [N] von der angewendeten Preßkraft [kN] für die Beispiele 1 bis 4 im Vergleich zu dem Standard.
Abbildung 2 zeigt die Abhängigkeit der Bruchfestigkeiten [N] von der angewendeten Preßkraft [kN] für die Beispiele 2, 5 und 6 sowie den Standard.
Abbildung 3 zeigt die Biegefestigkeit [N] für die Beispiele 7 bis 11.
Abbildung 4 zeigt die Wasseraufnahme [%] für die Beispiele 20 bis 23 während eines Zeitraums von 7 Tagen
Abbildung 5 zeigt die Abnahme der Tablettenhärte [%] für die Beispiele 20 bis 23.
Abbildung 6 zeigt die Abhängigkeit der Bruchfestigkeiten [N] von der angewendeten Preßkraft [kN] für die Beispiele 20 sowie 27 bis 30.
Abbildung 7 zeigt die Abhängigkeit der Zerfallszeit [min] von der Bruchfestigkeit [N] für die Beispiele 20 sowie 27 bis 30; die obere Grenze für eine akzeptable Zerfallszeit ist eingetragen. Die vom Europäischen Arzneibuch geforderte Obergrenze von 15 Minuten sollte weit unterschritten werden, um zu garantieren, dass stets alle Tabletten die geforderten Grenzen einhalten. Aus diesem Grund wird eine Zerfallszeit von weniger als 10 Minuten als akzeptabel beurteilt.
Abbildung 8 zeigt die Freisetzungskinetik von Tabletten der Beispiele 28 und 30 nach 8 Wochen Lagerung unter feuchten Bedingungen. Der prozentuale Anteil an freigesetztem Wirkstoff ist gegen die Zeit aufgetragen.
Abbildung 9 zeigt die Abhängigkeit der Bruchfestigkeiten [N] von der angewendeten Preßkraft [kN] für auf verschiedene Weise hergestellte Tabletten der Zusammensetzung aus Beispiel 28.
Abbildung 10 zeigt die Abhängigkeit der Bruchfestigkeiten [N] von der angewendeten Preßkraft [kN] für auf verschiedene Weise hergestellte Tabletten der Zusammensetzung aus Beispiel 30.

### Beispiele

### 1. Enrofloxacin-Tabletten

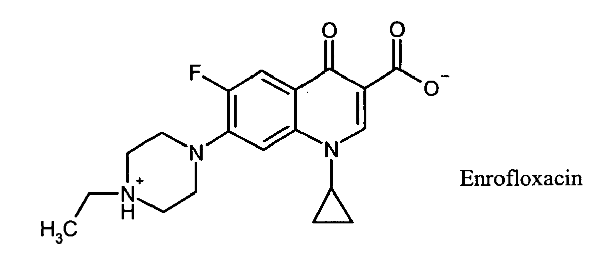

**Tabelle 1**

| Untersuchte Formulierungen: | | | | | |
|---|---|---|---|---|---|
| Tablettenzusammensetzung [mg/Tablette] | | | | | |
| | | Beispiel 1 | Beispiel 2 | Beispiel 3 | Beispiel 4 |
| Inhaltsstoff | Standard | 5% Bayopal | 10% Bayopal | 15% Bayopal | 10% Artificial Beef Flavor |
| Enrofloxacin | 50,0 | 50,0 | 50,0 | 50,0 | 50,0 |
| Lactose | 31,0 | 31,0 | 31,0 | 31,0 | 31,0 |
| Maisstärke | 27,0 | 27,0 | 27,0 | 27,0 | 27,0 |
| PVP 25 | 3,0 | 3,0 | 3,0 | 3,0 | 3,0 |
| Mikrokristalline Cellulose | 8,0 | 8,0 | 8,0 | 8,0 | 8,0 |
| Bayopal | | 6,0 | 12,0 | 18,0 | |
| Artificial Beef Flavor | | | | | 12,0 |
| Hochdisperses Siliciumdioxid | 0,2 | 0,2 | 0,2 | 0,2 | 0,2 |
| Magnesiumstearat | 0,8 | 0,8 | 0,8 | 0,8 | 0,8 |

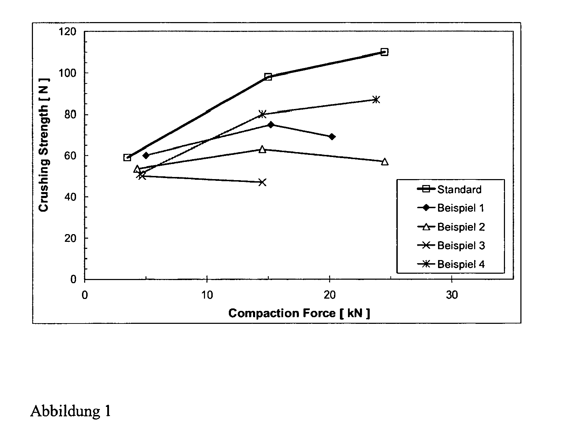

Durch den Zusatz des Aromastoffes wird die Tablettenhärte mit steigendem Anteil des Geschmacksstoffes herabgesetzt, wie die Abbildung 1 zeigt.

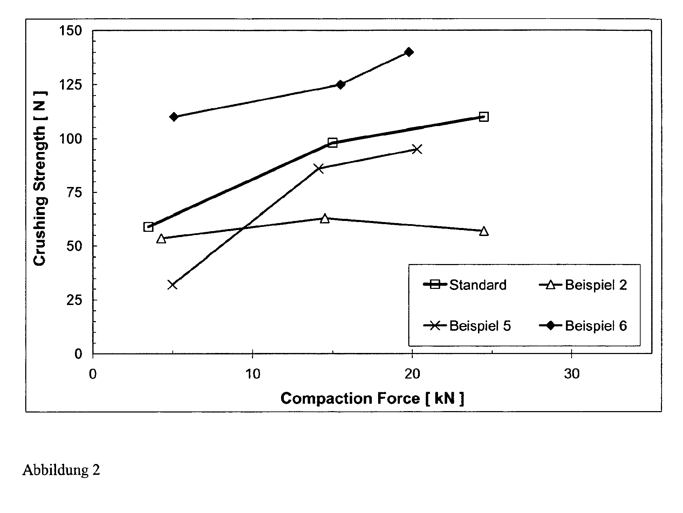

Durch Zusatz von hohen Mengen hochdispersem Siliciumdioxid (z.B. Aerosil 200®) sowie durch Zusatz weiterer Hilfsstoffe kann die Tablettenhärte verbessert werden (Abbildung 2).

**Tabelle 2**

| | | Beispiel 2 | Beispiel 5 | Beispiel 6 |
|---|---|---|---|---|
| Inhaltsstoff | Standard | 10% Bayopal | 10% Bayopal + 10% Aerosil | Zusätzliches MCC |
| Enrofloxacin | 50,0 | 50,0 | 50,0 | 50,0 |
| Lactose | 31,0 | 31,0 | 31,0 | 31,0 |
| Maisstärke | 27,0 | 27,0 | 27,0 | 27,0 |
| PVP 25 | 3,0 | 3,0 | 3,0 | 3,0 |
| Mikrokristalline Cellulose | 8,0 | 8,0 | 8,0 | 28,0 |
| Bayopal® | | 12,0 | 12,0 | 17,5 |
| Hochdisperses Siliciumdioxid | 0,2 | 0,2 | 12,2 | 10,0 |
| Croscarmellose Natrium | | | | 7,0 |
| Magnesiumstearat | 0,8 | 0,8 | 0,8 | 0,8 |

| | | | | |
|---|---|---|---|---|
| Mengenangaben der Inhaltsstoffe in mg/Tablette | | | | |

### II. Pradofloxacin-Tabletten

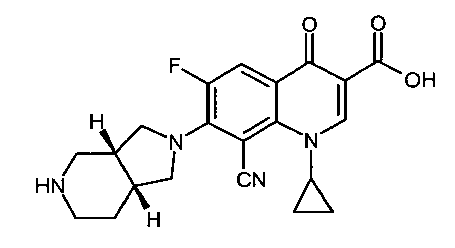

Für Pradofloxacin (Strukturformel) wurden verschiedene Formulierungsansätze gewählt und der Einfluss eines zugesetzten Aromastoffes auf die Tabletteneigenschaften geprüft.

**Tabelle 3**

| Mannit-basierte Formulierungen: | | | | | |
|---|---|---|---|---|---|
| | Beispiel 7 | Beispiel 8 | Beispiel 9 | Beispiel 10 | Beispiel 11 |
| Pradofloxacin | 100 | 100 | 100 | 100 | 100 |
| Mikrokristalline Cellulose | 20 | 20 | 20 | 20 | 20 |
| Mannit | 157,5 | 157,5 | 157,5 | 157,5 | 157,5 |
| PVP 25 | 15 | 15 | 15 | 15 | 15 |
| Artificial Beef Flavor | | | | | 15 |
| Bayopal® | | 15 | 15 | 15 | |
| Croscarmellose Natrium | 6 | 6 | 6 | 6 | 6 |
| Hochdisperses Siliciumdioxid | | | 15 | 3,75 | 15 |
| Magnesiumstearat | 1,5 | 1,5 | 1,5 | 1,5 | 1,5 |
| Verhältnis Biegefestigkeit/Presskraft | 2,13 x 10⁻³ | 2,00 x 10⁻³ | 3,03 10⁻ | 236 x 10⁻³ | 230 x 10⁻³ 3 |

| | | | | | |
|---|---|---|---|---|---|
| Mengenangaben der Inhaltsstoffe in mg/Tablette | | | | | |

Tablettenformat: oblong 14 x 6 r 5 [mm]

Durch Zusatz von Bayopal wird die Tablettenhärte (angegeben als Härteausbeute = Verhältnis Biegefestigkeit/Presskraft) herabgesetzt. Im Vergleich zu der aromastofffreien Formulierung geht die Biegefestigkeit der Oblongtabletten zurück. Durch Zusatz von hochdispersem Siliciumdioxid lässt sich die Tablettenhärte deutlich steigern, was Vorteile sowohl in der Fertigung (Transport, Verpackung) als auch bei der Applikation der Tabletten hat.

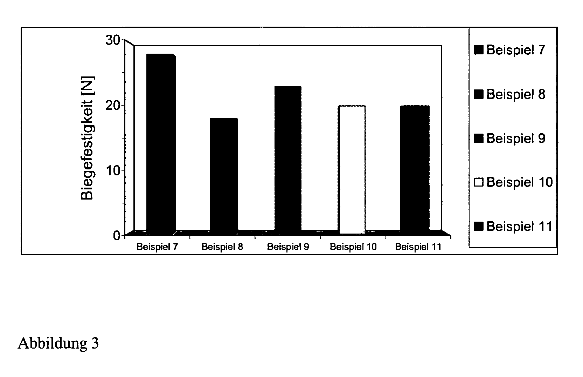

Zusätzliche Vorteile ergeben sich für die Formulierungen mit hochdispersem Siliciumdioxid bei Lagerung unter feuchten Bedingungen. Nach einer Woche Lagerung bei einer Luftfeuchtigkeit >80 % reduziert sich die Biegefestigkeit aller Tabletten mit Aromastoffen. Bei Einsatz hoher Mengen an hochdispersem Siliciumdioxid lässt sich dieser Rückgang, wie die folgende Abbildung 3 zeigt, auf ein für die Praxis akzeptables Maß reduzieren.

**Tabelle 4**

| Auf mikrokristalliner Cellulose basierende Formulierungen: | | | | | |
|---|---|---|---|---|---|
| | Beispiel 12 | Beispiel 13 | Beispiel 14 | Beispiel 15 | Beispiel 16 |
| Pradofloxacin | 100 | 100 | 100 | 100 | 100 |
| Mikrokristalline Cellulose | 152,8 | 152,8 | 152,8 | 152,8 | 152,8 |
| PVP 25 | 30 | 30 | 30 | 30 | 30 |
| Artificial Beef Flavor | | | | | 15 |
| Bayopal | | 15 | 15 | 15 | |
| Croscarmellose Natrium | 15 | 15 | 15 | 15 | 15 |
| Hochdisperses Siliciumdioxid | 0,9 | 0,9 | 15,9 | 4,65 | 15,9 |
| Magnesiumstearat | 1,35 | 1,35 | 1,35 | 1,35 | 1,35 |
| Verhältnis Biegefestigkeit/Presskraft | 6,53 x 10⁻³ | 4,25 x 10⁻³ | 5,29 x 10⁻³ | 4,68 x 10⁻³ | 6,46 x 10⁻³ |

| | | | | | |
|---|---|---|---|---|---|
| Mengenangaben der Inhaltsstoffe in mg/Tablette Tablettenformat: oblong 14 x 6 r 5 [mm] | | | | | |

Das bereits mehrfach beschriebene Prinzip der Verbesserung der Tablettenhärte gilt auch für Pradofloxacin-Formulierungen, die auf mikrokristalliner Cellulose basieren. Bei diesen Tabletten wirkt sich wie die vorstehende Tabelle zeigt der Zusatz von Aromastoffen besonders nachteilig auf die Tablettenhärte aus, was durch den Zusatz von hochdispersem Siliciumdioxid vermieden werden kann.

Pradofloxacin-Tabletten mit Lactose und Mikrokristalliner Cellulose:

Die folgende Mischung kann zu Tabletten unterschiedlicher Größe verpresst werden:

**Tabelle 5**

| | |
|---|---|
| Pradofloxacin | 15,0 % |
| Mikrokristalline Cellulose | 35,0 % |
| Milchzucker | 24,0 % |
| PVP 25 | 5,0 % |
| Artificial Beef Flavor | 10,0 % |
| Croscarmellose Natrium | 7,5 % |
| Hochdisperses Siliciumdioxid | 2,5 % |
| Magnesiumstearat | 1,0 % |

Tabletten mit folgenden Größen und Dosierungen sind beispielsweise möglich:

| | Dosis Pradofloxacin | Tablettenformat |
|---|---|---|
| Beispiel 17: | 15 mg | 8 x 4 r 4,5 [mm] |
| Beispiel 18: | 60 mg | 14 x 7 r 6 [mm] |
| Beispiel 19: | 120 mg | 18 x 8 r 6 [mm] |

Besonders empfindlich gegenüber Feuchtigkeit verhalten sich kleine Tabletten. Lagert man die Tabletten aus Beispiel 17 offen ohne schützende Verpackung bewahren sie eine für ihre Größe ausreichende Härte, was im folgenden dargestellt ist und die Befunde aus den vorherigen Pradofloxacin-Tabletten-Beispielen bestätigt und ergänzt.

**Tabelle 6**

| Tablettenhärte nach Offenlagerung bei 85% rel. Feuchte nach 15 Tagen: | |
|---|---|
| Presskraft [kN] | Tablettenhärte nach Offenlagerung [N] |
| 9 | 32 |
| 12 | 34 |
| 13,5 | 36 |

### III. Tanomastat-Tabletten

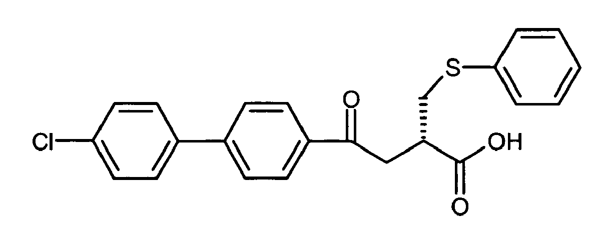

Tanomastat (Strukturformel) wurde in Form verschiedener geschmacksoptimierter Tabletten untersucht.

**Tabelle 7**

| Formulierungen mit Artificial Beef Flavor | | | | |
|---|---|---|---|---|
| | Beispiel 20 | Beispiel 21 | Beispiel 22 | Beispiel 23 |
| Tanomastat | 200,0 | 200,0 | 200,0 | 200,0 |
| Lactose | 121,20 | 121,20 | 121,20 | 121,20 |
| Mikrokristalline Cellulose | 60,8 | 60,80 | 60,80 | 60,80 |
| Na-laurylsulfat | 2,0 | 2,0 | 2,0 | 2,0 |
| Aritificial Beef Flavor | - | 40,0 | 40,0 | 20,0 |
| Croscarmellose Natrium | 12,0 | 12,0 | 12,0 | 12,0 |
| Hochdisp. Siliciumdioxid | - | - | 10,0 | 20,0 |
| Magnesiumstearat | 4,0 | 4,0 | 4,0 | 4,0 |

| | | | | |
|---|---|---|---|---|
| Mengenangaben der Inhaltsstoffe in mg/Tablette | | | | |

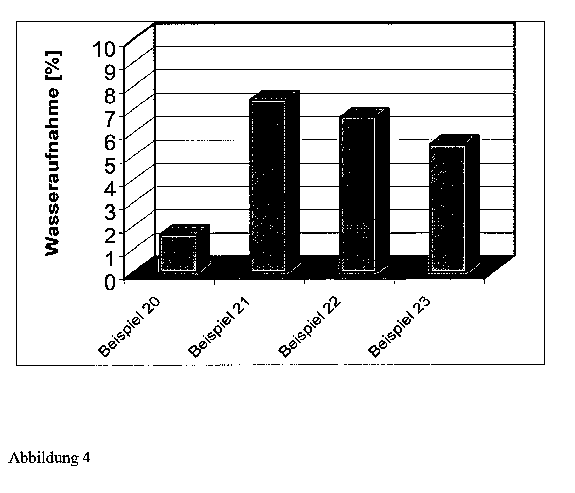

Beispiel 20 stellt als Vergleich eine Formulierung ohne Geschmacksstoff dar. Lagert man die geschmackstoffhaltigen Formulierungen unter feuchten Bedingungen, nehmen sie Wasser auf, was in der folgenden Abbildung 4 für die Beispiel-Formulierungen gezeigt ist. Durch die Zugabe von hochdispersem Siliciumdioxid in hohen Mengen lässt sich dies reduzieren.

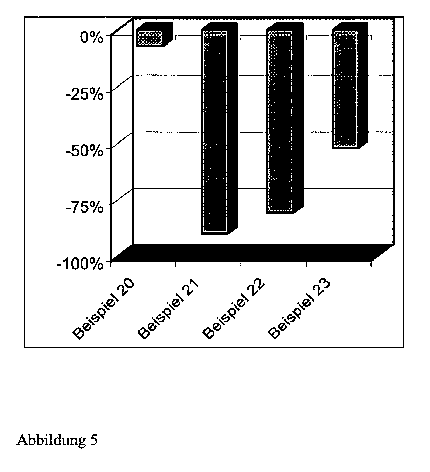

Während der Lagerung verlieren die Tabletten zusätzlich deutlich an Härte, was zu Problemen im Markt führen kann (Kundenreklamationen). Auch dieses Problem lässt sich durch die erfinderische Maßnahme verringern (siehe Abbildung 5).

Die Formulierung entsprechend Beispiel 23 kann zu Tabletten unterschiedlichster Größe verpresst werden, wie beispielsweise im folgenden dargestellt ist:

**Tabelle 8**

| | Beispiel 23 | Beispiel 24 | Beispiel 25 | Beispiel 26 |
|---|---|---|---|---|
| Tanomastat | 200,0 | 50,0 | 300,0 | 400,0 |
| Lactose | 121,20 | 30,3 | 181,8 | 242,4 |
| Mikrokristalline Cellulose | 60,80 | 15,2 | 91,2 | 121,6 |
| Na-laurylsulfat | 2,0 | 0,5 | 3,0 | 4,0 |
| Aritificial Beef Flavor | 20,0 | 5,0 | 30,0 | 40,0 |
| Croscarmellose Natrium | 12,0 | 3,0 | 18,0 | 24,0 |
| Hochdisp. Siliciumdioxid | 20,0 | 5,0 | 30,0 | 40,0 |
| Magnesiumstearat | 4,0 | 1,0 | 6,0 | 8,0 |
| Tablettenformat | 16*6 r 5 | 9*4 r 4,5 | 17*7 r 6 | 18*8 r 6 |

| | | | | |
|---|---|---|---|---|
| Mengenangaben der Inhaltsstoffe in mg/Tablette; Tablettenformat in mm | | | | |

Alle diese Formulierungen weisen die oben beschriebenen Vorteile bei feuchter Lagerung auf.

**Tabelle 9**

| Bayopal-haltige Formulierungen | | | | | |
|---|---|---|---|---|---|
| | Beispiel 20 | Beispiel 27 | Beispiel 28 | Beispiel 29 | Beispiel 30 |
| Tanomastat | 200,0 | 200,0 | 200,0 | 200,0 | 200,0 |
| Lactose | 121,20 | 121,20 | 121,20 | 121,20 | 121,20 |
| Mikrokristalline Cellulose | 60,80 | 60,80 | 60,80 | 60,80 | 60,80 |
| Na-laurylsulfat | 2,0 | 2,0 | 2,0 | 2,0 | 2,0 |
| Bayopal | - | 40,0 | 40,0 | 40,0 | 40,0 |
| Croscarmellose Natrium | 12,0 | 12,0 | 12,0 | 12,0 | 12,0 |
| Hochdisp. Siliciumdioxid | - | - | 40,0 | 20,0 | 10,0 |
| Magnesiumstearat | 4,0 | 4,0 | 4,0 | 4,0 | 4,0 |

| | | | | | |
|---|---|---|---|---|---|
| Mengenangaben der Inhaltsstoffe in mg/Tablette | | | | | |

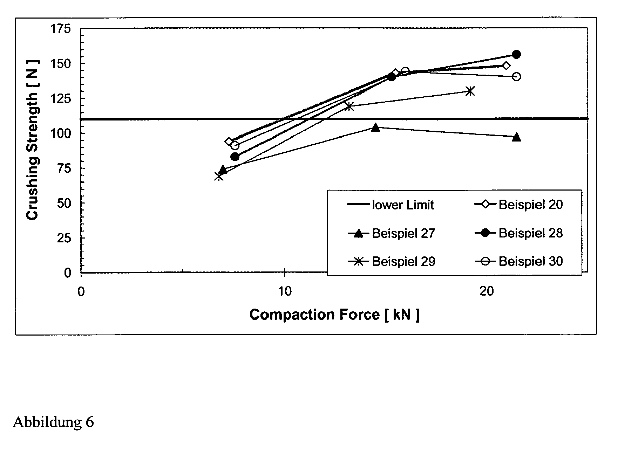

Durch den Zusatz von Bayopal verlieren die Formulierungen an Härte. Dies kann wie auch bereits für andere Wirkstoffe bzw. Geschmacksstoffe beschrieben durch den Zusatz von hochdispersem Siliciumdioxid vermieden werden (siehe Abbildung 6).

Neben dem negativen Einfluss auf die Tablettenhärte verschlechtert der Geschmacksstoff auch die Zerfalls- und Freisetzungseigenschaften. Auch diese negativen Auswirkungen lassen sich durch die beschriebene erfinderische Maßnahme vermindern.

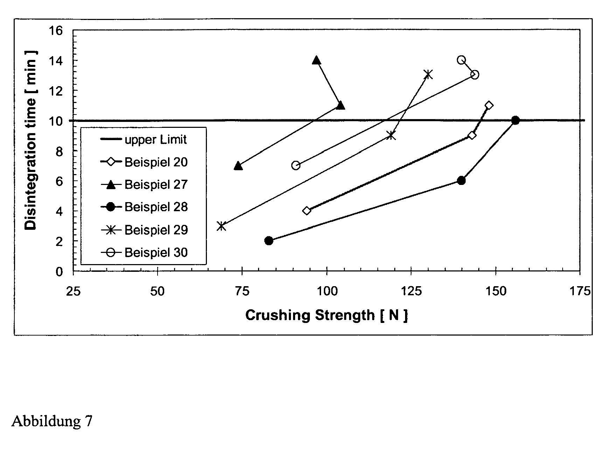

Der Zerfall der Tabletten mit hochdispersem Siliciumdioxid ist trotz der verbesserten Tablettenhärte schneller als bei Tabletten mit Geschmacksstoff ohne hochdisperses Siliciumdioxid (Abbildung 7).

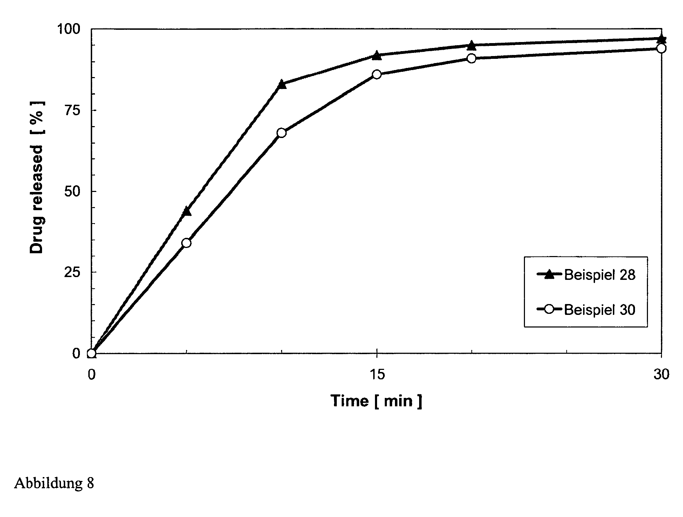

Nach 8 Wochen Lagerung unter feuchten Bedingungen zeigen Tabletten, die analog zur Zusammensetzung der Beispiele 28 und 30 als Tablette mit 50 mg Wirkstoff hergestellt wurden, eine verbesserte Freisetzung, wenn ein hoher Anteil an hochdispersem Siliciumdioxid eingearbeitet wurde (Abbildung 8).

### IV. Akzeptanzversuche bei Hunden

Die Formulierungen entsprechend Beispielen 23 und 24 wurden an 40 Hunden getestet. Im Gegensatz zu der in EP 00345787 bzw. US 04910023 beschriebenen Geschmacksmaskierung wurden die getesteten Formulierungen von 92,5% der untersuchten Hunde freiwillig aufgenommen, was belegt, dass der zugesetzte Aromastoff seine Funktionalität überraschenderweise nicht verloren hat.

### V. Einfluss des Herstellverfahrens

Für alle beschriebenen Formulierungen können verschiedene Herstellverfahren gewählt werden:
- Zusatz des Geschmacksstoffes und des hochdispersen Siliciumdioxids zur Nachmischung
- wässrige Granulation des Geschmacksstoffes mit dem hochdispersen Siliciumdioxid, Zusatz dieses Granulates zur Nachmischung oder zur weiteren Granulation
- alkoholische (z.B. ethanolische) Granulation des Geschmacksstoffes mit dem hochdispersen Siliciumdioxid, Zusatz dieses Granulates zur Nachmischung oder zur weiteren Granulation
- Granulation des Geschmacksstoffes mit dem hochdispersen Siliciumdioxid unter Verwendung eines weiteren Bindemittels, Zusatz dieses Granulates zur Nachmischung oder zur weiteren Granulation
- Zusatz des Geschmacksstoffes mit dem hochdispersen Siliciumdioxid zur Granulation des Wirkstoffes eventuell unter Zusatz weiterer Hilfsstoffe wie z.B. Füllmittel, Zerfallhilfsmittel oder Bindemittel

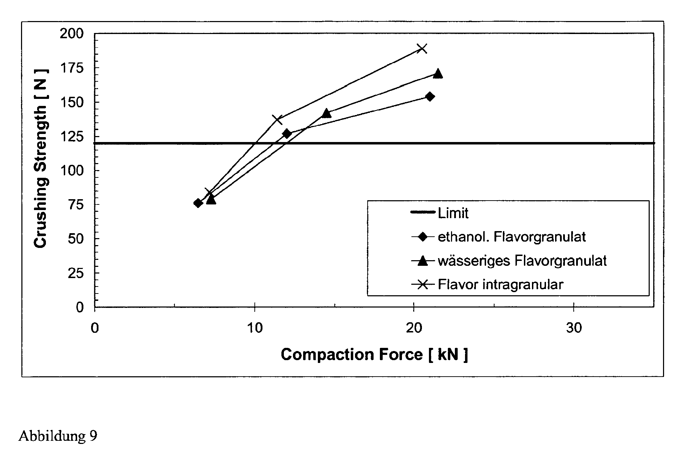

Diese Herstellungsverfahren führen zu Tabletten mit identischen Eigenschaften. In Abbildung 9 ist dies für die Härte von Tabletten entsprechend der Zusammensetzung aus Beispiel 28, die nach unterschiedlichen Granulationsverfahren hergestellt wurden, dargestellt.

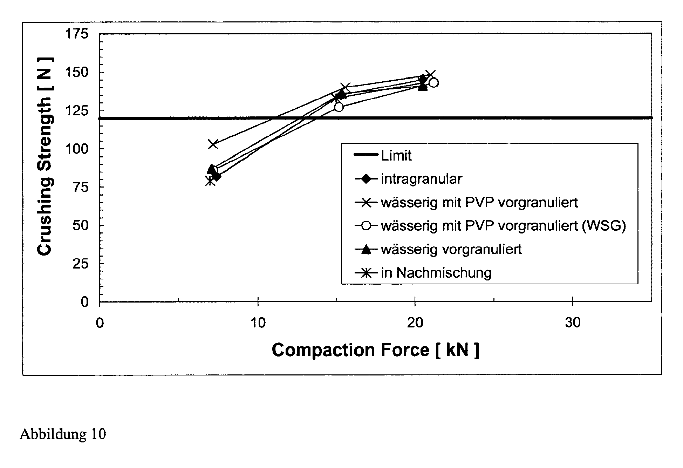

Wählt man für Tabletten gemäß Beispiel 30 unterschiedliche Herstellungsverfahren, werden ebenfalls Tabletten mit praktisch gleichen Presskraft/Härte-Profilen erhalten (Abbildung 10).

Diese Daten belegen, dass das Prinzip des Einarbeitens von hohen Mengen an hochdispersem Siliciumdioxid in geschmacksstoffhaltige Formulierungen zu einer Verbesserung der pharmazeutischen Eigenschaften von Tabletten führt, unabhängig vom gewählten Granulier- bzw. Tablettierverfahren.

## Patentansprüche

1. Feste Arzneimittelformulierung, enthaltend einen pharmazeutischen Wirkstoff, einen Aromastoff und/oder Geschmacksstoff und mindestens 1,5 Gew.-% hochdisperses Siliciumdioxid bezogen auf das Gesamtgewicht der fertigen Formulierung.

2. Feste Arzneimittelformulierung gemäß Anspruch 1, enthaltend als pharmazeutische Wirkstoffe Enrofloxacin, ein Enrofloxacinsalz oder ein Hydrat des Enrofloxacins oder seines Salzes.

3. Feste Arzneimittelformulierung gemäß Anspruch 1, enthaltend als pharmazeutischen Wirkstoff Pradofloxacin, ein Pradofloxacinsalz oder ein Hydrat des Pradofloxacins oder seines Salzes.

4. Verfahren zur Herstellung der festen Arzneimittelformulierung gemäß Anspruch 1 bei dem man den Geschmacksstoff mit dem hochdispersen Siliciumdioxid sowie einem oder mehreren Wirkstoffen und pharmazeutisch üblichen Zusatz- und/oder Hilfsstoffen granuliert.
